# EUROPEAN PATENT APPLICATION

(11) **EP 4 421 161 A1**
(43) Date of publication of application: **28.08.2024**
(21) Application number: 22900595.4
(22) Date of filing: 01.12.2022
(51) Int. Cl.: C12M 1/34, C12N 5/10

(54) **CONTROL SYSTEM FOR CELL THERAPY AND CONTROL METHOD THEREFOR**

(30) Priority: 01.12.2021 CN 202111457104
(71) Applicant: Nanjing GenScript Biotech Co., Ltd., Nanjing, Jiangsu 211100 (CN)
(72) Inventor: ZHANG, Hao, Nanjing, Jiangsu 211100 (CN); WANG, Lin, Nanjing, Jiangsu 211100 (CN); SHI, Gaofeng, Nanjing, Jiangsu 211100 (CN); QIAN, Hong, Nanjing, Jiangsu 211100 (CN)
(74) Representative: Wang, Bo
(86) International application number: PCT/CN2022/135828
(87) International publication number: WO 2023/098791

(57) **Abstract**

A control system for cell therapy and a control method therefor. The control system may comprise a cell processing module, an auxiliary connection module, and a cell culture module which are connected in sequence; and a processor which is in communication connection with the cell processing module, the auxiliary connection module and the cell culture module, wherein the processor controls the auxiliary connection module to temporarily store a cell solution of the cell processing module and to transfer the temporarily stored cell solution to the cell culture module.

## Description

### Claim of Priority

This application is a continuation of International Application No. PCT/CN2022/135828, filed on December 1, 2022, which claims priority to Chinese Patent Application No. 202111457104.1, filed on December 1, 2021, the entire contents of each of which are incorporated herein by reference.

### Technical Field

This specification relates to the field of cell processing systems, and in particular, to a control system for cell therapy and a control method therefor.

### Background Art

In recent years, with the development of technology, cell therapy has become a popular field of research and development in the biomedical industry. The cell therapy has a relatively complex process in which a variety of cell processing devices are involved during preparation of a target cell product. The cell processing devices are independent of each other, such that when an operator transfers a cell solution of a cell processing device according to corresponding steps, it is necessary to manually transfer the cell solution to other cell processing devices. This manual transfer method is low in efficiency, and frequent transfer of the cell solution by the operator increases the risk of contamination.

Therefore, the present application provides a control system for cell therapy capable of providing a closed environment and operating automatically and a control method therefor.

### Summary of the Invention

An embodiment of this specification provides a control system for cell therapy, including: a cell processing module, an auxiliary connection module, and a cell culture module which are connected in sequence; and a processor which is in communication connection with the cell processing module, the auxiliary connection module and the cell culture module, wherein the processor controls the auxiliary connection module to temporarily store a cell solution of the cell processing module and to transfer the temporarily stored cell solution to the cell culture module.

An embodiment of this specification provides a control method for a control system for cell therapy, the control system including a cell processing module, an auxiliary connection module, and a cell culture module which are connected in sequence, the control method including: controlling the cell processing module to pretreat a cell solution, and transferring and temporarily storing the pretreated cell solution in the auxiliary connection module; controlling the auxiliary connection module to temporarily store the cell solution of the cell processing module and to transfer the temporarily stored cell solution to the cell culture module; and controlling the cell culture module to culture the cell solution.

### Brief Description of the Drawings

This specification will be further illustrated by way of exemplary embodiments, and these exemplary embodiments will be described in detail with reference to the drawings. These embodiments are not restrictive, and in these embodiments, the same numerals represent the same structures. In the drawings:
FIG. 1 is a schematic diagram of an exemplary module structure of a control system for cell therapy according to some embodiments of this specification;
FIG. 2 is a schematic diagram of an exemplary structure of a control system according to some embodiments of this specification;
FIG. 3 is a schematic diagram of an exemplary control panel of a control system according to some embodiments of this specification;
FIG. 4 is a schematic diagram of an exemplary structure of a control system according to some embodiments of this specification;
FIG. 5 is a schematic diagram showing an exemplary pipeline principle of an auxiliary connection module according to some embodiments of this specification;
FIG. 6 is an exemplary flowchart of a control method for a control system for cell therapy according to some embodiments of the present application;
FIG. 7 is a flowchart of solution transfer by controlling an auxiliary connection module according to some embodiments of the present application.

### Detailed Description of Embodiments

For a clearer description of the technical solutions in the embodiments of this specification, the drawings required for describing the embodiments will be briefly described below. Apparently, the drawings in the following description show merely some examples or embodiments of this specification, and those of ordinary skill in the art may still apply this specification to other similar scenarios according to these drawings without any creative effort. Unless apparent from the context or otherwise stated, the same reference signs in the drawings represent the same structure or operation.

It should be understood that the term "system", "apparatus", "unit", and/or "module" used herein refer to a method used to distinguish different levels of different components, elements, parts, portions, or assemblies. However, these words may be substituted by other expressions if these expressions can accomplish the same purpose.

As shown in this specification and the claims, the words "one", "a", "an" and/or "the" do not specifically refer to the singular, but may also include the plural, unless the context clearly indicates otherwise. Generally, the terms "comprise" and "include" only imply the inclusion of explicitly identified steps and elements, and these steps or elements do not constitute an exclusive list. A method or a device may further include other steps or elements.

Flowcharts are used in this specification to illustrate operations performed by a system according to the embodiments of this specification. It should be understood that preceding or following operations are not necessarily performed in an exact order. Instead, all steps may be executed in a reverse order or simultaneously. Moreover, it is possible to add other operations to these processes, or remove one or more operations from these processes.

In the process of cell therapy, a variety of cell processing devices are involved during preparation of a target cell product. Because the cell processing devices are independent of each other, after a cell solution is treated by a cell processing device, it is necessary to measure corresponding parameters of the cell solution before the cell solution can enter a next cell processing device for treatment. During this process, in order to ensure physiological activities of the cell solution, the cell solution needs to be manually collected and stored during measurement of the corresponding parameters. The manual operation is low in efficiency, and the cell solution has a high risk of contamination after many transfers, causing the resulting cell products to fail to meet expected requirements.

In this specification, a control system for cell therapy is mainly described. The control system for cell therapy mainly includes a cell processing module, an auxiliary connection module, and a cell culture module which are connected in sequence, wherein the auxiliary connection module connects the cell processing module to the cell culture module, so as to create a closed environment for the treatment and/or culture of the cell solution, which can isolate the cell solution from the outside throughout the cell therapy process, thereby reducing the risk of contamination of the cell solution. The cell processing module can be used to perform treatment (e.g., cell washing, cell incubation and cell sorting) on an initial cell solution, the auxiliary connection module can be used to transfer the treated cell solution of the cell processing module to the cell culture module, and the cell culture module can be used to perform culture (e.g., cell activation, cell transduction and cell expansion) on the treated cell solution. The operations, such as manual transfer of the cell solution, throughout the process of cell therapy are reduced, which, on one hand, reduces the risk of contamination of the cell solution, and on the other hand, reduces the workload of an operator and improves the efficiency of cell therapy. In addition, the auxiliary connection module can also be used to temporarily store the cell solution to facilitate the operator's subsequent operations on the cell solution, such as the measurement of corresponding parameters of the cell solution. The cell processing module and/or the cell culture module can be flexibly combined and mounted by means of the auxiliary connection module according to different cell processing processes, so as to make combination and control of the control system more flexible.

In some embodiments, the control system for cell therapy may further include a processor which is in communication connection with the cell processing module, the auxiliary connection module and the cell culture module. The processor may separately control the cell processing module, the auxiliary connection module and the cell culture module, so as to control operating states of the cell processing module, the auxiliary connection module and the cell culture module to achieve the effect of automatic operation. It should be noted that the control system for cell therapy is not only applicable to a preparation process for cell therapy, but also to other cell-related processes (e.g., a cell processing process and a cell culture process, etc.).

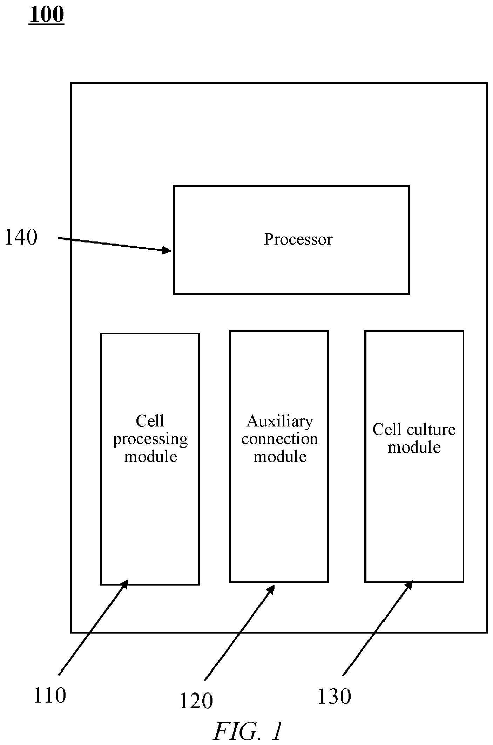

FIG. 1 is a schematic diagram of an exemplary module structure of a control system 100 for cell therapy according to some embodiments of this specification. As shown in FIG. 1, the control system 100 for cell therapy may include a cell processing module 110, an auxiliary connection module 120 (also referred to as an auxiliary connection apparatus), a cell culture module 130, and a processor 140 which are connected in sequence.

The cell processing module 110 can be used to perform pretreatment on a cell solution (e.g., washing treatment, incubation treatment, and/or sorting treatment of the cell solution). In some embodiments, the cell processing module 110 may include one or more of a cell washing unit, a cell incubation unit and a cell sorting unit. The cell washing unit is a device for removing impurities in the cell solution by washing. In some embodiments, the cell washing unit may be a centrifuge. The cell incubation unit is a device for incubating cells in the cell solution. In some embodiments, the cell incubation unit 213 may be a cell incubator. The cell sorting unit is a device for separating target cells from the cell solution. The treatment units in the cell processing module 110 may be selected according to the type of the cell solution to be treated. For example, when a cell solution obtained by a manufacturer is a cell solution that has been treated by washing and incubation, the cell processing module 110 may include a cell sorting unit. For another example, when a cell solution obtained by a manufacturer is a cell solution that has been treated by washing, the cell processing module 110 may include a cell incubation unit and a cell sorting unit. For a further example, when a cell solution obtained by a manufacturer is an entirely untreated cell solution, the cell processing module 110 may include a cell washing unit, a cell incubation unit and a cell sorting unit. When the cell processing module 110 includes a plurality of treatment units, the plurality of treatment units may also be connected by means of the auxiliary connection module 120.

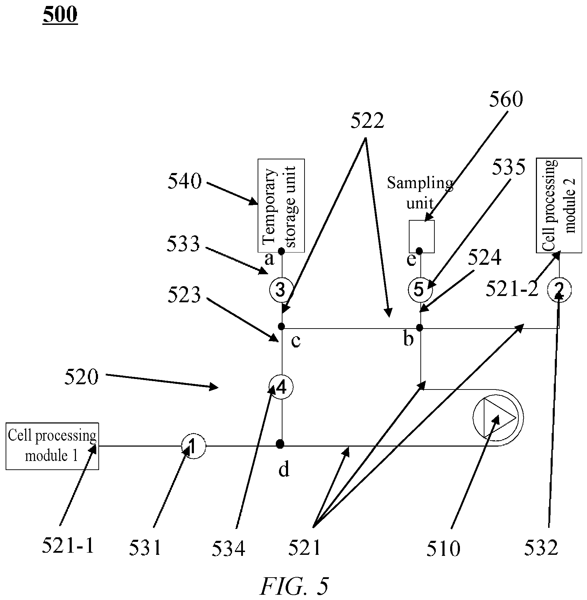

The auxiliary connection module 120 is configured to connect the cell processing module 110 to the cell culture module 130. Specifically, the auxiliary connection module 120 may be configured to collect and temporarily store the cell solution treated by the cell processing module 110, and when the cell solution temporarily stored in the auxiliary connection module 120 meets a corresponding condition, the temporarily stored cell solution may be transferred to the cell culture module 130. In some embodiments, the auxiliary connection module 120 may include a solution transfer pipeline assembly, a power unit and a temporary storage unit, and the cell processing module 110 and the cell culture module 130 may be connected to each other by means of a first solution transfer pipeline in the solution transfer pipeline assembly. The temporary storage unit is configured to collect and temporarily store the cell solution treated by the cell processing module, and the temporary storage unit is connected to the first solution transfer pipeline by means of a second solution transfer pipeline in the solution transfer pipeline assembly. The power unit is adapted to the first solution transfer pipeline assembly and provides power drive for the transfer of the cell solution. In some embodiments, the auxiliary connection module 120 may further include a heat preservation unit, and the temporary storage unit is located in the heat preservation unit. The heat preservation unit provide the cell solution in the temporary storage unit with a specific temperature (e.g., 2°C to 8°C), thereby ensuring the physiological activities of cells in the temporary storage unit. In some embodiments, the auxiliary connection module 120 may further include a sampling unit connected to the first solution transfer pipeline by means of a fourth solution transfer pipeline of the solution transfer pipeline assembly. The sampling unit may sample the cell solution treated by the cell processing module 110, thereby facilitating detection of sample parameter information of the cell solution, so as to determine whether the cell solution temporarily stored in the temporary storage unit can be transferred to the cell culture module 130. It should be noted that the auxiliary connection module 120 is not only used for connection between the cell processing module 110 and the cell culture module 130, but also for connection between corresponding treatment units (e.g., the cell washing unit, the cell incubation unit and the cell sorting unit) in the cell processing module 110 and connection between corresponding units in the cell culture module 130. In some embodiments, one or more auxiliary connection modules 120 may be provided. The specific number of auxiliary connection modules 120 may be determined according to the number of corresponding treatment units in the cell processing module 110, the number of corresponding treatment units in the cell culture module 130, and the number of other devices. In order to facilitate distinction and description of different auxiliary connection modules 120, they are described below as a first auxiliary connection module, a second auxiliary connection module, a third auxiliary connection module and a fourth auxiliary connection module. The details of the auxiliary connection modules 120 can be provided with reference to other parts of this specification, such as FIG. 5 and the related description.

The cell culture module 130 can perform activation, transduction, expansion and other operations on the cell solution. In some embodiments, the cell culture module 130 may include one or more of a cell activation unit, a cell transduction unit and a cell expansion unit. The cell activation unit is a device for promoting cell division. In some embodiments, the cell activation unit may be a cell activation device to which a cell activator (e.g., activating magnetic beads and interleukin-2 growth factor. etc.) is added. The cell transduction unit is a device that can perform a transduction operation on cells in the cell solution to enable target DNA/RNA to enter the cells to obtain target cells. In some embodiments, the cell transduction unit may be an electroporator. The cell expansion unit is a device that can perform culture and expansion on the target cells in the cell solution. In some embodiments, the cell expansion unit may be a cell expansion instrument. A culture unit in the cell culture module 130 may be selected according to the type of the cell solution to be cultured. For example, when the cell solution delivered by the auxiliary connection module 120 is a cell solution after a cell activation step and a cell transduction step, the cell culture module 130 may include a cell expansion unit. For another example, when the cell solution delivered by the auxiliary connection module 120 is a cell solution after a cell activation step, the cell culture module 130 may include a cell transduction unit and a cell expansion unit. For another example, when the cell solution delivered by the auxiliary connection module 120 is a cell solution only treated by the cell processing module 110, the cell culture module 130 may include a cell activation unit, a cell transduction unit and a cell expansion unit. When the cell culture module 130 includes a plurality of culture units, the plurality of culture units may be connected to each other by means of the auxiliary connection module 120. In some embodiments, the cell activation unit, the cell transduction unit and the cell expansion unit may be integrated into one device, or may be devices independent of each other.

The processor 140 may control operating states of the cell processing module 110, the auxiliary connection module 120 and the cell culture module 130. In some embodiments, the processor 140 may be in communication connection with the cell processing module 110, the auxiliary connection module 120 and the cell culture module 130, so as to respectively control the cell processing module 110, the auxiliary connection module 120 and the cell culture module 130. For example, the processor 140 may control the cell processing module 110 to pretreat the cell solution. When the cell processing module 110 includes one or more treatment units, the processor 140 may control the treatment units (e.g., the cell washing unit, the cell incubation unit, and the cell sorting unit) to correspondingly treat the cell solution. For another example, the processor 140 may control, based on the end status of each treatment unit, operation of the auxiliary connection module 120 to perform solution transfer, temporarily storage, sampling, etc. on the pretreated cell solution.

The operator may detect sample parameter information of the cell solution based on the cell solution in the sampling unit, and then input the sample parameter information into the control system 100 for cell therapy. In some embodiments, the processor 140 may determine, based on the sample parameter information and preset parameter information preset in the control system 100 for cell therapy, whether the sample parameter information of the cell solution temporarily stored in the auxiliary connection module 120 satisfies the preset parameter information. If the sample parameter information satisfies the preset parameter information, the processor 140 enables the cell solution temporarily stored in the auxiliary connection module 120 to be transferred to the cell culture module 130. If the sample parameter information does not satisfy the preset parameter information, the processor 140 may send corresponding instructions, and control the system to send corresponding prompt information to enable the operator to pretreat the cell solution again.

In some embodiments, the sample parameter information may include, but is not limited to, at least one of the volume, cell density and cell viability of the cell solution. The cell viability refers to a proportion of living cells in total cells in the cell solution. The cell density is the number of cells per unit cell solution. After obtaining the sample parameter information of the cell solution, the operator inputs the sample parameter information of the cell solution into the control system 100 for cell therapy. In some embodiments, the preset parameter information may include a preset cell viability range and/or a preset cell number range. The processor 140 may determine whether the preset parameter information is satisfied based on the sample parameter information of the cell solution temporarily stored in the auxiliary connection module 120. For example, the processor 140 may determine whether the number of cells in the cell solution temporarily stored in the auxiliary connection module 120 is within the preset cell number range, or the processor 140 may determine whether the cell viability of the cell solution temporarily stored in the auxiliary connection module 120 is within the preset cell viability range. If the sample parameter information satisfies the preset parameter information, the processor 140 enables the cell solution temporarily stored in the auxiliary connection module 120 to be transferred to the cell culture module 130. If the sample parameter information does not satisfy the preset parameter information, the processor 140 may send corresponding instructions, and control the system to send corresponding prompt information to enable the operator to pretreat the cell solution again. In some embodiments, the processor 140 may calculate the number of cells in the cell solution temporarily stored in the auxiliary connection module 120 according to the volume and the cell density of the cell solution.

In some embodiments, the processor 140 may be integrated in some devices of the control system 100. For example, the processor 140 may be integrated in the auxiliary connection module 120. For another example, the processor 140 may be integrated in the cell processing module 110. For still another example, the processor 140 may be integrated in the cell culture module 130. In some embodiments, the processor 140 may be a single server or a server group. The server group may be centralized or distributed. In some embodiments, the processor 140 may be a local component or a remote component relative to one or more other components of the control system 100. For example, the processor 140 may access information and/or data stored in the cell processing module 110, the auxiliary connection module 120, and the cell culture module 130 via a network (e.g., a cable network, a fiber-optic network, a telecommunication network, the Internet, or any combination thereof). As another example, the processor 140 may be directly connected to the cell processing module 110, the auxiliary connection module 120 and the cell culture module 130 to access the stored information and/or data. In some embodiments, the processor 140 may be implemented on a cloud platform. Only as an example, the cloud platform may include a private cloud, a public cloud, a hybrid cloud, a community cloud, a distributed cloud, an internal cloud, a multi-layer cloud, or any combination thereof. In some embodiments, the processor 140 may include a central treatment unit (CPU), an application-specific integrated circuit (ASIC), an application-specific instruction-set processor (ASIP), a graphics treatment unit (GPU), a physics treatment unit (PPU), a digital signal processor (DSP), a field programmable gate array (FPGA), a programmable logic device (PLD), a controller, a microcontroller unit, a reduced instruction set computer (RISC), a microprocessor, etc., or any combination thereof.

It should be noted that the above description is provided for illustrative purposes only and is not intended to limit the scope of the present application. Many changes and modifications can be made by those skilled in the art under the guidance of the content of the present application. The features, structures, methods and other features of the exemplary embodiments described in the present application may be combined in various manners to obtain additional and/or alternative exemplary embodiments. However, these changes and modifications do not depart from the scope of the present application.

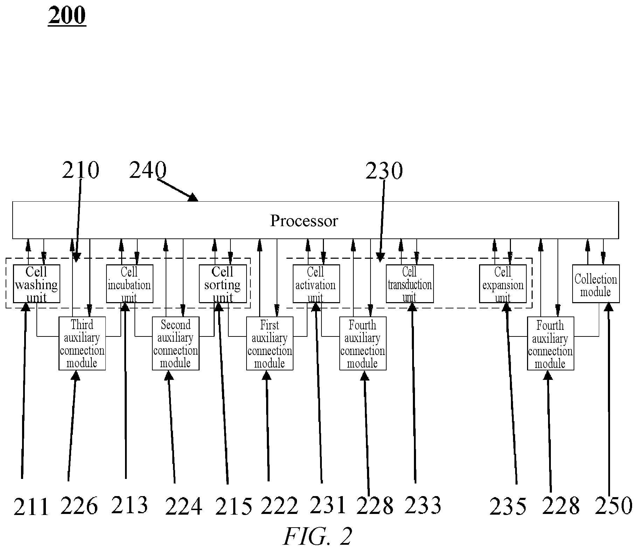

FIG. 2 is a schematic diagram of an exemplary structure of a control system 200 according to some embodiments of this specification.

As shown in FIG. 2, in some embodiments, the control system 200 may include a cell processing module 210, an auxiliary connection module and a cell culture module 230, and the auxiliary connection apparatus may include a first auxiliary connection module 222, wherein the cell processing module 210 is connected to the cell culture module 230 by means of the first auxiliary connection module 222. The auxiliary connection module in one or more embodiments of this specification may also be referred to as an auxiliary connection apparatus.

In some embodiments, the cell processing module 210 may include a cell washing unit 211, a cell incubation unit 213 and a cell sorting unit 215, which are arranged sequentially.

The cell washing unit 211, the cell incubation unit 213 and the cell sorting unit 215 may be connected to each other by means of auxiliary connection modules. In some embodiments, the auxiliary connection modules may include a first auxiliary connection module 222, a second auxiliary connection module 224 and a third auxiliary connection module 226. An output end of the cell washing unit 211 is connected to an input end of the cell incubation unit 213 by means of the third auxiliary connection module 226, an output end of the cell incubation unit 213 is connected to an input end of the cell sorting unit 215 by means of the second auxiliary connection module 224, and an output end of the cell sorting unit 215 is connected to an output end of the cell culture module 230 by means of the first auxiliary connection module 222, such that the modules and units are connected in sequence. During the operation of the control system 200, the cell solution undergoes washing treatment by the cell washing unit 211, the cell solution is then transferred through the third auxiliary connection module 226 to the cell incubation unit 213 for incubation treatment and then transferred through the second auxiliary connection module 224 to the cell sorting unit 215 for sorting treatment, and the sorted cell solution is transferred through the first auxiliary connection module 222 to the cell culture module 230 for culture.

In some embodiments, two or more cell processing modules 210 may be provided, the two or more cell processing modules 210 are connected to the first auxiliary connection module 222, and the plurality of cell processing modules 210 are arranged in parallel. In some embodiments, when a plurality of cell processing modules 210 may be provided, corresponding treatment units in two or more cell processing modules 210 may be the same or different, which may be specifically selected according to actual production situations.

It should be noted that treatment units in the cell processing module 210 are not limited to the cell washing unit 211, cell incubation unit 213 and cell sorting unit 215 mentioned above, and may be adjusted accordingly according to a cell therapy process. For example, treatment units are added or omitted. For example, if the initial cell solution has undergone cell washing, the cell processing module 210 may include a cell incubation unit 213 and a cell sorting unit 125 connected in sequence, and the cell incubation unit 213, the second auxiliary connection module 224, the cell sorting unit 215, the first auxiliary connection module 222 and the cell culture module 230 are connected in sequence, and the cell washing unit 211 and the third auxiliary connection module 226 may be omitted. For another example, if the initial cell solution has undergone cell washing and cell incubation, the cell processing module 210 may include only a cell sorting unit 215, and the cell sorting unit 215, the first auxiliary connection module 222 and the cell culture module 230 are connected in sequence while the cell washing unit 211, the cell incubation unit 213, the third auxiliary connection module 226 and the second auxiliary connection module 224 may be omitted.

In some embodiments, the cell culture module 230 may include a cell activation unit 231, a cell transduction unit 233 and a cell expansion unit 235.

In some embodiments, the cell activation unit 231, the cell transduction unit 233 and the cell expansion unit 235 may be integrated into one apparatus, and the cell solution pretreated by the cell processing module 210 may enter the apparatus and then sequentially undergo activation, transduction and expansion steps. In some embodiments, the cell activation unit 231, the cell transduction unit 233 and the cell expansion unit 235 may alternatively be three different apparatuses which are connected by means of auxiliary connection modules. The cell solution sequentially enters the cell activation unit 231, the cell transduction unit 233 and the cell expansion unit 235 to complete the activation, transduction and expansion steps. In some embodiments, the cell activation unit 231, the cell transduction unit 233 and the cell expansion unit 235 may alternatively be two different apparatuses. For example, the cell activation unit 231 is a single apparatus, and the cell transduction unit 233 and the cell expansion unit 235 are another apparatus; or the cell activation unit 231 and the cell transduction unit 233 are integrated into one apparatus, and the cell expansion unit 235 is another separate apparatus.

In some embodiments, the control system 200 may further include a collection module 250 for collecting a target product, and the auxiliary connection module may further include a fourth auxiliary connection module 228, wherein the cell culture module 230 is connected to the collection module 250 by means of the fourth auxiliary connection module 228, and the cell solution that has been cultured in the cell culture module 230 is transferred through the fourth auxiliary connection module 228 to the collection module 250 for collection. In some embodiments, the operator can further sample, through the fourth auxiliary connection module 228, the cell solution in the cell culture module 230 for detection and analysis. In some embodiments, the fourth auxiliary connection module 228 for sampling and the fourth auxiliary connection module 228 for transferring the cell solution may be the same or different. In some embodiments, the collection module 250 may be a liquid filling device. In some embodiments, the collection module 250 may be a container for holding a cell solution, such as a solution bag and a solution tube, etc.

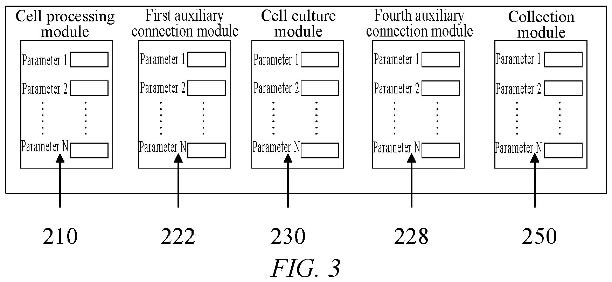

In order to provide a clearer description of the specific operating process of the control system 200, the control system 200 is further described with reference to FIG. 3. FIG. 3 is a schematic diagram of an exemplary control panel of a control system 200 according to some embodiments of this specification.

With reference to FIGS. 2 and 3, after the cell solution enters the cell washing unit through an input end of the cell washing unit 211, the operator may input relevant parameter information (e.g., parameter 1, parameter 2, ..., and parameter N shown in the cell processing module 210 in FIG. 3) in a control panel of the control system 200, and the processor 240 controls the cell washing unit 211 to perform cell washing treatment according to the parameter information. In some embodiments, the relevant parameter information may include one or more of an operating mode of the cell washing unit 211 (e.g., an on-off state and a rotation speed of the cell washing unit 211), an initial volume of the cell solution (the volume of the cell solution entering the cell washing unit 211), a target volume of the cell solution (the volume of the cell solution after cell washing treatment), a washing time, etc. The relevant parameter information herein may be preset or temporarily input. After the cell washing unit 211 finishes the washing of the cell solution, the processor 240 controls the third auxiliary connection module 226 to perform temporarily storage, heat preservation, sampling and other treatment on the cell solution based on the end status of the cell washing unit 211. At this time, the cell solution is discharged from the output end of the cell washing unit 211 under the action of the third auxiliary connection module 226 and enters the temporary storage unit of the third auxiliary connection module 226 for heat preservation and temporary storage, and at the same time, part of the solution in the cell washing unit 211 enters the sampling unit for sampling. The operator performs sampling and analysis on the cell solution in the third auxiliary connection module 226 to obtain sample parameter information of the cell solution, and inputs the sample parameter information of the cell solution into the control panel. In some embodiments, the sample parameter information may include the volume, cell viability and cell density of the cell solution, and the processor 240 may calculate the number of cells based on the input volume and cell density of the cell solution. The cell viability refers to a proportion of living cells in total cells in the cell solution. The cell density is the number of cells per unit cell solution. In some embodiments, the processor 240 may determine whether the sample parameter information of the cell solution temporarily stored in the third auxiliary connection module 226 satisfies preset parameter information preset in the control system 200. In some embodiments, the preset parameter information includes a preset cell viability range and/or a preset cell number range. When the number of cells in the cell solution temporarily stored in the third auxiliary connection module 226 is within the preset cell number range and the cell viability is within the preset cell viability range, the processor 240 sends a signal to control the third auxiliary connection module 226 to transfer the temporarily stored cell solution to the cell incubation unit 213, and the cell solution is discharged from an output end of the third auxiliary connection module 226 and enters the cell incubation unit 213 through the input end of the cell incubation unit 213.

After the cell solution enters the cell incubation unit 213, the operator may input relevant parameter information (e.g., parameter 1, parameter 2, ..., and parameter N shown in the cell processing module 210 in FIG. 3) into the control panel of the control system 200, and the processor 240 sends a signal according to the relevant parameter information to control the cell incubation unit 213 to perform cell incubation treatment. In some embodiments, the relevant parameter information may include one or more of an operating mode of the cell incubation unit 213 (e.g., an on-off state and a temperature range of the cell incubation unit 213, etc.), an initial volume of the cell solution (the volume of the cell solution entering the cell incubation unit 213 from the third auxiliary connection module 226), a target volume of the cell solution (the volume of the cell solution after cell incubation treatment), the incubation time, etc. The relevant parameter information herein may be preset or temporarily input. After the cell incubation unit 213 finishes the incubation of the cell solution, the processor 240 controls the second auxiliary connection module 224 to perform temporarily storage, heat preservation, sampling and other treatment on the cell solution based on the end status of the cell incubation unit 213. At this time, the cell solution is discharged from the output end of the cell incubation unit 213 under the action of the second auxiliary connection module 224 and enters the temporary storage unit of the second auxiliary connection module 224 for heat preservation and temporary storage, and at the same time, part of the solution in the cell incubation unit 213 may enter the sampling unit for sampling. The operator performs sampling and analysis on the cell solution in the second auxiliary connection module 224 to obtain sample parameter information of the cell solution, and inputs the sample parameter information of the cell solution into the control panel. In some embodiments, the processor 240 may determine whether the sample parameter information of the cell solution temporarily stored in the second auxiliary connection module 224 satisfies the preset parameter information preset in the control system 200. When the number of cells in the cell solution temporarily stored in the second auxiliary connection module 224 is within the preset cell number range and the cell viability is within the preset cell viability range, the processor 240 sends a signal to control the second auxiliary connection module 224 to transfer the temporarily stored cell solution to the cell sorting unit 215, and the cell solution is discharged from an output end of the second auxiliary connection module 224 and enters the cell sorting unit 215 through the input end of the cell sorting unit 215.

After the cell solution enters the cell sorting unit 215, the operator may input relevant parameter information (e.g., parameter 1, parameter 2, ..., and parameter N shown in the cell processing module 210 in FIG. 3) into the control panel of the control system 200, and the processor 240 sends a signal according to the relevant parameter information to control the cell sorting unit 215 to perform cell sorting treatment. In some embodiments, the relevant parameter information may include one or more of an operating mode of the cell sorting unit 215 (an on-off state, etc. of the cell sorting unit 215), an initial volume of the cell solution (the volume of the cell solution entering the cell sorting unit 215 from the second auxiliary connection module 224), a target volume of the cell solution (the volume of the cell solution after cell sorting treatment), a sorting time, etc. The relevant parameter information herein may be preset or temporarily input. After the cell sorting unit 215 finishes the sorting of the cell solution, the processor 240 controls the first auxiliary connection module 222 to perform temporarily storage, heat preservation, sampling and other treatment on the cell solution based on the end status of the cell sorting unit 215. At this time, the cell solution is discharged from the output end of the cell sorting unit 215 under the action of the first auxiliary connection module 222 and enters the temporary storage unit of the first auxiliary connection module 222 for heat preservation and temporary storage, and at the same time, part of the solution in the cell sorting unit 215 enters the sampling unit for sampling. The operator performs sampling and analysis on the cell solution in the first auxiliary connection module 222 to obtain sample parameter information of the cell solution, and inputs the sample parameter information of the cell solution into the control panel. In some embodiments, the processor 240 may determine whether the sample parameter information of the cell solution temporarily stored in the first auxiliary connection module 222 satisfies the preset parameter information preset in the control system 200. When the number of cells in the cell solution temporarily stored in the first auxiliary connection module 222 is within the preset cell number range and the cell viability is within the preset cell viability range, the processor 240 sends a signal to control the first auxiliary connection module 222 to transfer the temporarily stored cell solution to the cell culture module 230, and the cell solution is discharged from an output end of the first auxiliary connection module 222 and enters the cell culture module 230 through an input end of the cell culture module 230.

In some embodiments, the cell activation unit 231, the cell transduction unit 233 and the cell expansion unit 235 are integrated into one apparatus. After the cell solution enters the cell culture module 230, the processor 240 sends a signal to control the cell culture module 230 to perform cell activation treatment. After the cell culture module 230 finishes the activation of the cell solution, the processor 240 controls the fourth auxiliary connection module 228 to sample the cell solution based on the status of end of activation of the cell culture module 230. At this time, a small amount of cell solution is discharged from the output end of the cell culture module 230 under the action of the fourth auxiliary connection module 228 and enters the sampling unit of the fourth auxiliary connection module 228 for sampling. The operator performs sampling and analysis on the cell solution in the fourth auxiliary connection module 228 to obtain sample parameter information of the cell solution, and inputs the sample parameter information of the cell solution into the control panel. In some embodiments, the processor 240 may determine whether the sample parameter information of the cell solution temporarily stored in the fourth auxiliary connection module 228 satisfies the preset parameter information preset in the control system 200. When the number of cells in the cell solution temporarily stored in the fourth auxiliary connection module 228 is within the preset cell number range and the cell viability is within the preset cell viability range, the processor 240 sends a signal to control the cell culture module 230 to perform the cell transduction step.

After the cell culture module 230 finishes the transduction of the cell solution, the processor 240 sends, based on the status of end of cell transduction of the cell culture module 230, a signal to control the cell culture module 230 to perform the cell expansion step.

After the cell culture module 230 finishes the expansion of the cell solution, the processor 240 controls the fourth auxiliary connection module 228 to perform temporarily storage, heat preservation, sampling and other treatment on the cell solution based on the status of end of cell expansion of the cell culture module 230. At this time, the cell solution is discharged from the output end of the cell culture module 230 under the action of the fourth auxiliary connection module 228 and enters the temporary storage unit of the fourth auxiliary connection module 228 for heat preservation and temporary storage, and at the same time, part of the solution in the cell culture module 230 enters the sampling unit for sampling. The operator performs sampling and analysis on the cell solution in the fourth auxiliary connection module 228 to obtain sample parameter information of the cell solution, and inputs the sample parameter information of the cell solution into the control panel. In some embodiments, the processor 240 may determine whether the sample parameter information of the cell solution temporarily stored in the fourth auxiliary connection module 228 satisfies the preset parameter information preset in the control system 200. When the number of cells in the cell solution temporarily stored in the fourth auxiliary connection module 228 is within the preset cell number range and the cell viability is within the preset cell viability range, the processor 240 sends a signal to control the fourth auxiliary connection module 228 to transfer the temporarily stored cell solution to the collection module 250 to complete collection, and the cell solution is discharged from an output end of the fourth auxiliary connection module 228 and enters the collection module 250 through an input end of the collection module 250.

After the cell solution enters the collection module 250, the staff may input relevant parameter information (e.g., parameter 1, parameter 2, ..., and parameter N shown in the collection module 250 in FIG. 3) into the control panel of the control system 200, and the processor 240 sends a signal according to the relevant parameter information to control the collection module 250 to perform collection treatment. In some embodiments, the relevant parameter information may include one or more of an operating mode of the collection module 250 (e.g., an on-off state of the collection module 250, etc.), an initial volume of the cell solution (the volume of the cell solution entering the collection module 250), the quantity and volume to be dispensed, etc. The relevant parameter information herein may be preset or temporarily input. After the collection module 250 finishes the dispensing and collection of the cell solution, the processor 240 sends a signal based on the end status of the collection module 250, and the control system sends corresponding prompt information.

In some embodiments, the cell activation, cell transduction and cell expansion in the cell culture module 230 may be sequentially performed in the cell activation unit 231, the cell transduction unit 233 and the cell expansion unit 235 arranged independently of each other. In this case, the cell activation unit 231, the cell transduction unit 233 and the cell expansion unit 235 are connected to each other by means of auxiliary connection modules.

It should be noted that in the above cell culture process, if any sample parameter information does not satisfy the preset parameter information, the cell culture will be stopped immediately at the corresponding step, and the cell solution will be discarded and the pipeline will be replaced for the next use. In addition, FIGS. 2 and 3 and the related contents are only an example in this specification, and one or more modules or units may be omitted or added in specific applications. For example, the collection module 250 may be omitted. For another example, the cell washing unit 211 in the cell processing module 210 may be omitted.

In some embodiments, sample parameter information input by the units and/or modules may be the same or different. For example, sample parameter information input by the third auxiliary connection module 226 may be the cell viability and the total number of cells in the cell solution, sample parameter information input by the second auxiliary connection module 224 may include the cell viability and the cell density, and sample parameter information input by the first auxiliary connection module 222 may be the cell viability, the cell density, the total number of cells, etc.

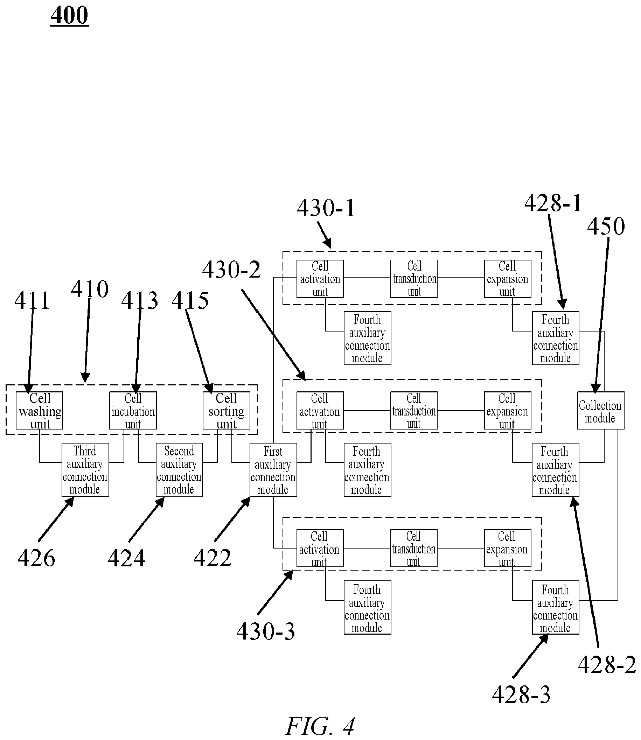

In the process of cell culture, it takes a shorter time (e.g., 1-12 h) to perform cell processing, while it takes a longer time (e.g., 3-20 days) to perform the cell expansion step. Therefore, there is a long time during which the cell culture module cannot be put into use, and the cell processing module and the auxiliary connection module are idle, reducing the working efficiency. On this basis, in some embodiments, the control system may include a plurality of cell culture modules, and the plurality of cell culture modules are arranged in parallel. With the arrangement of the plurality of cell culture modules, when one of the cell culture modules is used for cell therapy, the other cell culture modules can still be used, such that the control system as a whole will not be idle, thus greatly improving the efficiency of cell culture. For details, reference is made to FIG. 4 and the related description.

FIG. 4 is a schematic diagram of an exemplary structure of a control system 400 according to some embodiments of this specification.

Referring to FIG. 4, in some embodiments, a plurality of cell culture modules may be provided, the plurality of cell culture modules are connected to auxiliary connection modules, and the plurality of cell culture modules are arranged in parallel. For example, the control system 400 may include a cell processing module 410, an auxiliary connection module, a cell culture module 430-1, a cell culture module 430-2 and a cell culture module 430-3. The cell processing module 410 may include a cell washing unit 411, a cell incubation unit 413 and a cell sorting unit 415. The auxiliary connection modules include a first auxiliary connection module 422, a second auxiliary connection module 424, a third auxiliary connection module 426, a fourth auxiliary connection module 428-1, a fourth auxiliary connection module 428-2 and a fourth auxiliary connection module 428-3. The cell washing unit 411, the third auxiliary connection module 426, the cell incubation unit 413, the second auxiliary connection module 424, the cell sorting unit 415 and the first auxiliary connection module 422 are connected in sequence. The cell culture module 430-1, the cell culture module 430-2 and the cell culture module 430-3 are all connected to the first auxiliary connection module 422. The cell culture module 430-1 is connected to the collection module 450 by means of the fourth auxiliary connection module 428-1, the cell culture module 430-2 is connected to the collection module 450 by means of the fourth auxiliary connection module 428-2, and the cell culture module 430-3 is connected to the collection module 450 by means of the fourth auxiliary connection module 428-3. Under the control of the processor, only one cell culture module is in communication with the first auxiliary connection module 422 at a time.

After the cell solution enters the cell washing unit 411, the processor controls the cell washing unit 411 to perform cell washing treatment on the cell solution. The processor receives information of the end of cell washing, and controls the third auxiliary connection module 426 to perform heat preservation, sampling and temporary storage on the cell solution. The operator performs sampling and analysis on the cell solution to obtain sample parameter information and inputs the sample parameter information into the control system 400. The processor receives a signal of the sample parameter information satisfying the preset parameter information, and controls the third auxiliary connection module 426 to transfer the temporarily stored cell solution to the cell incubation unit 413.

After the cell solution enters the cell incubation unit 413, the processor controls the cell incubation unit 413 to perform cell incubation treatment on the cell solution. The processor receives information of the end of cell incubation, and controls the second auxiliary connection module 424 to perform heat preservation, sampling and temporary storage on the cell solution. The operator performs sampling and analysis on the cell solution to obtain sample parameter information and inputs the sample parameter information into the control system 400. The processor receives a signal of the sample parameter information satisfying the preset parameter information, and controls the second auxiliary connection module 424 to transfer the temporarily stored cell solution to the cell sorting unit 415.

After the cell solution enters the cell sorting unit 415, the processor controls the cell sorting unit 415 to perform cell sorting treatment on the cell solution. The processor receives information of the end of cell sorting, and controls the first auxiliary connection module 422 to perform heat preservation, sampling and temporary storage on the cell solution. The operator performs sampling and analysis on the cell solution to obtain sample parameter information and inputs the sample parameter information into the control system 400. The processor receives a signal of the sample parameter information satisfying the preset parameter information, and controls the first auxiliary connection module 422 to transfer the temporarily stored cell solution to the cell culture module 430-1.

At this time, the cell culture module 430-1 is occupied for cell expansion and other operations. The cell processing module 410 may be put into use again. The above steps are repeated, and finally the processor controls the first auxiliary connection module 422 to transfer the temporarily stored cell solution to the cell culture module 430-2 or 430-3. This improves the efficiency of cell culture and improve the usage efficiency of the control system 400.

After the cell solution enters the cell culture modules 430-1, 430-2 and 430-3, the processor controls the cell culture modules 430-1/430-2/430-3 to perform culture, such as cell activation, cell transduction and cell expansion, on the cell solution. After receiving information of the end of cell culture, the processor controls the corresponding fourth auxiliary connection modules 428-1, 428-2 and 428-3 to perform heat preservation, sampling and temporary storage on the cell solution. The operator performs sampling and analysis on the cell solution to obtain sample parameter information and inputs the sample parameter information into the control system 400. The processor receives a signal of the sample parameter information satisfying the preset parameter information, and controls the corresponding fourth auxiliary connection modules 428-1, 428-2 and 428-3 to transfer the temporarily stored cell solution to the collection module 450. For the specific steps of cell culture and the operating process of the cell processing module 410 and the cell culture module 430, reference can be made to FIGS. 2 and 3 and the related description.

It should be noted that it is only for illustrative purposes herein, and the number of cell culture modules is not limited to three shown in FIG. 4, but may be two, four, five or more, and may be specifically determined depending on an actual situation.

In some embodiments, after the cell processing module 410 is used once, it may be necessary to clean and replace some of the pipelines of the control system 400 (i.e., the pipeline before the first auxiliary connection module 422) to reduce the risk of contamination of the cell solution due to liquid residue.

In some embodiments, the control system 400 may alternatively include a plurality of cell processing modules 410 and a plurality of cell culture modules. The plurality of cell processing modules 410 are arranged in parallel and are all connected to one first auxiliary connection module 422, and the plurality of cell culture modules are arranged in parallel and are all connected to one first auxiliary connection module 422. The operating process of the plurality of cell processing modules 410 is similar to that of the plurality of cell culture modules described above, which will not be repeated herein.

FIG. 5 is a schematic diagram showing an exemplary pipeline principle of an auxiliary connection module 500 according to some embodiments of this specification. Referring to FIG. 5, in some embodiments, the auxiliary connection module 500 may include a power unit 510 and a solution transfer pipeline assembly 520 adapted to the power unit 510.

The power unit 510 is mainly configured to provide a power source for the flow of the cell solution to enable the cell solution to flow in the solution transfer pipeline assembly 520. In some embodiments, the power unit 510 may be a peristaltic pump, and the solution transfer pipeline assembly 520 is adapted to a pump head of the peristaltic pump. In some embodiments, the portion of the pipeline in the solution transfer pipeline assembly 520 that is adapted to the pump head of the peristaltic pump is a hose disposed between the pump head and a housing of the peristaltic pump. The peristaltic pump can accurately pump a small amount of the cell solution, and the peristaltic pump does not come into contact with the cell solution during pumping of the cell solution, while the cell solution is in contact with only the outer side of the pipeline of the solution transfer pipeline assembly 520, thus achieving the aseptic and clean transfer effect.

In some embodiments, the solution transfer pipeline assembly 520 may include one or more of a rigid tube, a flexible tube, etc. Preferably, for more convenient and flexible installation, the solution transfer pipeline assembly 520 can use a flexible tube. In some embodiments, in order to facilitate the operator to observe the transfer of the cell solution, the solution transfer pipeline assembly 520 may use a transparent tube.

With continued reference to FIG. 5, in some embodiments, the solution transfer pipeline assembly 520 may include a first solution transfer pipeline 521. The first solution transfer pipeline 521 may include a first port 521-1 and a second port 521-2. The first port 521-1 may be connected to the cell processing module, and the second port 521-2 may be connected to the cell culture module, so as to implement the transfer of the solution between the cell processing module and the cell culture module. The power unit 510 is adapted to the first solution transfer pipeline 521 to provide power for the transfer of the solution from the cell processing module to the cell culture module.

In some embodiments, the auxiliary connection module 500 may further include a temporary storage unit 540 connected to the solution transfer pipeline assembly 520. The temporary storage unit 540 is mainly configured to temporarily store the cell solution treated by the cell processing module, so as to facilitate the operator's subsequent operations, such as heat preservation of the cell solution. In some embodiments, the temporary storage unit 540 may include one or more of a solution bag, a solution tank, etc., as long as it has good sealing performance and does not react with the stored cell solution, which is not excessively limited in this specification.

In some embodiments, the solution transfer pipeline assembly 520 may further include a second solution transfer pipeline 522 (section a-c-b shown in FIG. 5) for providing a channel for the cell solution to enter the temporary storage unit 540, and the second solution transfer pipeline 522 communicates the temporary storage unit 540 with the first solution transfer pipeline 521. Specifically, one end of the second solution transfer pipeline 522 is connected to the temporary storage unit 540, and the other end of the second solution transfer pipeline 522 is connected to the first solution transfer pipeline 521. The junction (point b shown in FIG. 5) of the first solution transfer pipeline 521 and the second solution transfer pipeline 522 is located between the second port 521-2 and the power unit 510. Since the cell solution needs to be pumped by the power unit 510 before power can be provided for the cell solution to flow in the solution transfer pipeline assembly 520, the junction b of the first solution transfer pipeline 521 and the second solution transfer pipeline 522 is located between the second port 521-2 and the power unit 510, such that the cell solution enters the first solution transfer pipeline 521 from the first port 521-1 and then finally enters, through the second solution transfer pipeline 522, the temporary storage unit 540 for storage.

In some embodiments, the solution transfer pipeline assembly 520 may further include a third solution transfer pipeline 523 (section c-d shown in FIG. 5) for providing a channel for the cell solution to discharge from the temporary storage unit 540, and the third solution transfer pipeline 523 is in communication with the second solution transfer pipeline 522 and the first solution transfer pipeline 521. A junction (point d shown in FIG. 5) of the third solution transfer pipeline 523 and the first solution transfer pipeline 521 is located between the first port 521-1 and the power unit 510. Since the cell solution needs to be pumped by the power unit 510 such that power can be provided for the cell solution to flow in the solution transfer pipeline assembly 520, the junction d of the third solution transfer pipeline 523 and the first solution transfer pipeline 521 is located between the first port 521-1 and the power unit 510. Under the action of the power unit 510, the cell solution in the temporary storage unit 540 enters the first solution transfer pipeline 521 through part of the second solution transfer pipeline 522 (i.e., the second solution transfer pipeline 522 between the temporary storage unit 540 and the third solution transfer pipeline 523) and the third solution transfer pipeline 523, and then enters the cell culture module.

In order to facilitate the control of a flow direction of the solution, in some embodiments, a valve may be provided on a solution transfer pipeline corresponding to the solution transfer pipeline assembly 520. In some embodiments, the first solution transfer pipeline 521 between the first port 521-1 and the power unit 510 is provided with a first valve 531, and the first valve 531 is configured to control the connection or disconnection of the first solution transfer pipeline 521 between the first port 521-1 and the power unit 510. The first solution transfer pipeline 521 between the second port 521-2 and the power unit 510 is provided with a second valve 532, and the second valve 532 is configured to control the connection or disconnection of the first solution transfer pipeline 521 between the second port 521-2 and the power unit 510. The second solution transfer pipeline 522 is provided with a third valve 533, and the third valve 533 is configured to control the connection or disconnection of the second solution transfer pipeline 522 between the first solution transfer pipeline 521 and the temporary storage unit 540. The third solution transfer pipeline 523 is provided with a fourth valve 534, and the fourth valve 534 is configured to control the connection or disconnection of the third solution transfer pipeline 523 between the first solution transfer pipeline 521 and the second solution transfer pipeline 522.

In order to provide a clearer description of the solution transfer pipeline assembly 520 in the auxiliary connection module, the description is provided below with reference to different delivery situations of the solution. When the solution is directly transferred from the cell processing module to the cell culture module, the first valve 531 and the second valve 532 are opened, and the third valve 533 and the fourth valve 534 are closed. Under the action of the power unit 510, the solution is discharged from the cell processing module, enters the first solution transfer pipeline 521 from the first port 521-1, and is then discharged from the second port 521-2 to enter the cell culture module.

When the solution is transferred from the cell processing module to the temporary storage unit 540, the first valve 531 and the third valve 533 are opened, and the second valve 532 and the fourth valve 534 are closed. Under the action of the power unit 510, the solution is discharged from the cell processing module, enters the first solution transfer pipeline 521 from the first port 521-1, enters the second solution transfer pipeline 522 through part of the first solution transfer pipeline 521 (i.e., the first solution transfer pipeline 521 between the cell processing module and the second solution transfer pipeline 522), and then enters the temporary storage unit 540.

When the solution is transferred from the temporary storage unit 540 to the cell culture module, the second valve 532, the third valve 533 and the fourth valve 534 are opened, and the first valve 531 is closed. Under the action of the power unit 510, the solution is discharged from the temporary storage unit 540, enters the first solution transfer pipeline 521 through part of the second solution transfer pipeline 522 (i.e., the second solution transfer pipeline 522 between the temporary storage unit 540 and the third solution transfer pipeline 523) and the third solution transfer pipeline 523, and then enters the cell culture module.

In order to facilitate detection of a previously treated cell solution (e.g., the cell solution treated by the cell processing module), in some embodiments, the auxiliary connection module may further include a sampling unit 560, and the solution transfer pipeline assembly 520 may further include a fourth solution transfer pipeline 524 (section e-b shown in FIG. 5), and the sampling unit 560 is in communication with the first solution transfer pipeline 521 by means of the fourth solution transfer pipeline 524. Under the action of the power unit 510, the solution in the solution cell processing module enters the fourth solution transfer pipeline 524 through part of the first solution transfer pipeline 521 (i.e., the first solution transfer pipeline 521 between the cell processing module and the fourth solution transfer pipeline 524) and flows to the sampling unit 560. The operator may take out the solution from the sampling unit 560 to sample the solution for subsequent operations, such as detection and analysis, and sample storage.

In some embodiments, the sampling unit 560 may be detachably arranged relative to the fourth solution transfer pipeline 524, so as to facilitate the operator to directly remove the sampling unit 560 for subsequent operations. The detachable connection herein may be snap-fit connection, bonding, threaded connection, etc. In some embodiments, the sampling unit 560 may be fixedly arranged, and correspondingly, the sampling unit 560 is provided with a sampling hole that can be opened and closed, so that the operator can extract, through the sampling hole, the solution from the sampling unit 560 for subsequent operations. In some embodiments, the sampling unit 560 may include, but is not limited to, a solution bag, a solution tank, a test tube, a liquid storage tank, and other containers.

In some embodiments, since the solution needs to be pumped by the power unit 510 such that power can be provided for the solution to flow in the solution transfer pipeline assembly 520, a junction of the fourth solution transfer pipeline 524 and the first solution transfer pipeline 521 is located between the second port 521-1 and the power unit 510. Under the action of the power unit 510, the solution enters the first solution transfer pipeline 521, then enters the fourth solution transfer pipeline 524 through part of the first solution transfer pipeline 521 (i.e., the first solution transfer pipeline 521 between the cell processing module and the fourth solution transfer pipeline 524), and finally enters the sampling unit 560.

In some embodiments, the fourth solution transfer pipeline 524 is provided with a fifth valve 535, and the fifth valve 535 is configured to control the connected and disconnected state of the fourth solution transfer pipeline 524. In some embodiments, the fifth valve 535 may be a pinch valve, and the fifth valve 535 is located outside the fourth solution transfer pipeline 524. For details of the fifth valve 535, reference can be made to the related description of the first valve 531 and other valves mentioned above, which will not be repeated herein.

When part of the solution is transferred from the cell processing module to the sampling unit 560, the first valve 531 and the fifth valve 535 are opened, and the second valve 532, the third valve 533 and the fourth valve 534 are closed. The solution is discharged from the cell processing module, enters the first solution transfer pipeline 521 from the first port 521-1, is pumped by the first valve 531 and the power unit 510, then flows through the first solution transfer pipeline 521 to enter the fourth solution transfer pipeline 524, passes through the fifth valve 535, and then enters the sampling unit 560 from the fourth solution transfer pipeline 524.

Since the sampling unit 560 is mainly used to sample the solution to facilitate subsequent operations, the capacity of the sampling unit 560 is relatively small, and occupies a small proportion of the whole solution, thus having little influence on solution transfer. Therefore, in some embodiments, when the solution is transferred out of the cell processing module, the solution can be transferred to the sampling unit 560 and the temporary storage unit 540 at the same time. In this case, the first valve 531, the third valve 533 and the fifth valve 535 are opened, and the second valve 532 and the fourth valve 534 are closed. The solution is discharged from the cell processing module, enters the first solution transfer pipeline 521 from the first port 521-1, is pumped by the first valve 531 and the power unit 510, then flows through the first solution transfer pipeline 521, and then enters the second solution transfer pipeline 522 and the fourth solution transfer pipeline 524 at the same time. The solution entering the second solution transfer pipeline 522 flows through the third valve 533 and then enters the temporary storage unit 540. The solution entering the fourth solution transfer pipeline 524 flows through the fifth valve 535 and then enters the sampling unit 560.

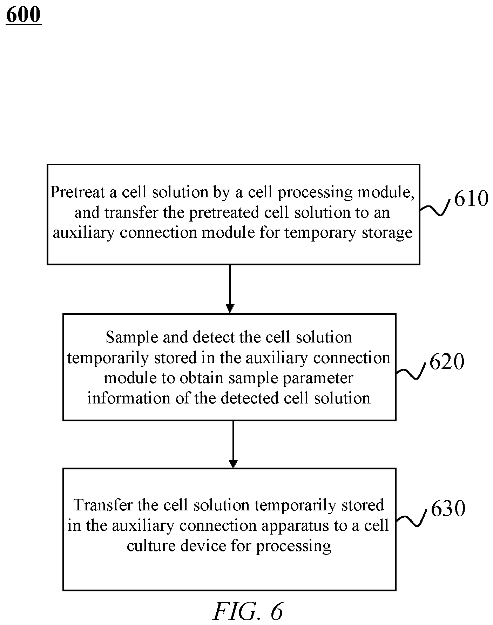

FIG. 6 is an exemplary flowchart of a control method for a control system for cell therapy according to some embodiments of the present application. As shown in FIG. 6, a process 600 may include the following steps.

In step 610, a cell processing module is controlled to pretreat a cell solution, and the pretreated cell solution is transferred to and temporarily stored in an auxiliary connection module.

The cell solution may be a solution containing target cells. In some embodiments, the cell solution may be an untreated cell solution. In this case, the cell processing module may include a cell washing unit, a cell incubation unit and a cell sorting unit, and the cell processing module may perform cell washing treatment, cell incubation treatment and cell sorting treatment on the cell solution. In some embodiments, the cell solution may alternatively be a cell solution treated by cell washing. In this case, the cell processing module may include a cell incubation unit and a cell sorting unit, and the cell processing module may perform cell incubation treatment and cell sorting treatment on the cell solution. In some embodiments, the cell solution may alternatively be a cell solution treated by cell washing and cell incubation. In this case, the cell processing module may include a cell sorting unit, and the cell processing module may perform cell sorting treatment on the cell solution.

After the cell solution enters the cell processing module, the processor controls the cell processing module to treat the cell solution. The processor receives a signal of the end of pretreatment of the cell solution, and controls the auxiliary connection module to transfer the pretreated cell solution. For the operating process of the cell processing module, reference is made to FIGS. 2 and 3, which will not be repeated herein.

In some embodiments, the auxiliary connection module may include a temporary storage unit, and the auxiliary connection module enables the pretreated cell solution to be transferred to and temporarily stored in the temporary storage unit. In order to ensure the physiological activities of the cells in the temporary storage unit, in some embodiments, the auxiliary connection module may include a heat preservation unit which can provide an environment with a preset temperature (e.g., 2°C to 8°C) for the cell solution in the temporary storage unit. In some embodiments, the auxiliary connection module may include a sampling unit which can sample the cell solution pretreated by the cell processing module, so as to obtain sample parameter information of the cell solution pretreated by the cell processing module. In order to ensure the physiological activities of the cells in the sampling unit, the sampling unit is also located in the heat preservation unit. For details about the treatment and transfer of the cell solution by the cell processing module and the treatment unit thereof, reference can be made to FIGS. 2 and 3 and the related description. For details about the auxiliary connection module, reference is made to the related description of FIG. 5, which will not be repeated herein.

In step 620, the auxiliary connection module is controlled to temporarily store the cell solution of the cell processing module and to transfer the temporarily stored cell solution to a cell culture module.

In some embodiments, the processor receives a signal of the end of pretreatment of the cell solution, and controls the auxiliary connection module to transfer the pretreated cell solution to the auxiliary connection module for temporary storage. Then, the operator may perform sampling and analysis on the cell solution in the sampling unit to obtain sample parameter information of the cell solution (e.g., the cell density, cell viability, and volume of the cell solution, etc.), and input the sample parameter information into the control system 100. In some embodiments, the sampling unit may be connected to an external device for measuring sample parameter information, and the device may automatically obtain and measure the sample parameter information of the cell solution in the sample unit based on the control of the processor. After receiving the sample parameter information, the processor may further determine whether the sample parameter information satisfies preset parameter information (e.g., the cell viability and the number of cells). When the sample parameter information satisfies the preset parameter information, the processor controls the auxiliary connection module to transfer the temporarily stored cell solution to the cell culture module. For details of the comparison between the sample parameter information and the preset parameter information, reference is made to FIGS. 2 and 3 and the related description.

In step 630, the cell culture module is controlled to culture the cell solution.

In some embodiments, the cell culture module may include at least one of a cell activation unit, a cell transduction unit and a cell expansion unit. The processor may control the cell culture module to sequentially perform corresponding culture (e.g., cell activation, cell transduction, and cell expansion) on the cell solution. After the processor receives a signal of the end of cell culture, the processor controls the auxiliary connection module to transfer the cell solution. For details of the operating process of the cell culture module, reference is made to FIGS. 2 and 3 and the related description.

It should be noted that the above description of the process 600 is only for illustration and explanation, and does not limit the scope of application of the present application. For those skilled in the art, various modifications and changes can be made to the process 600 under the guidance of the present application. However, these modifications and changes are still within the scope of the present application. For example, the pretreatment process of the cell solution in step 610 is divided into multiple steps of cell washing treatment, cell incubation treatment and/or cell sorting treatment.

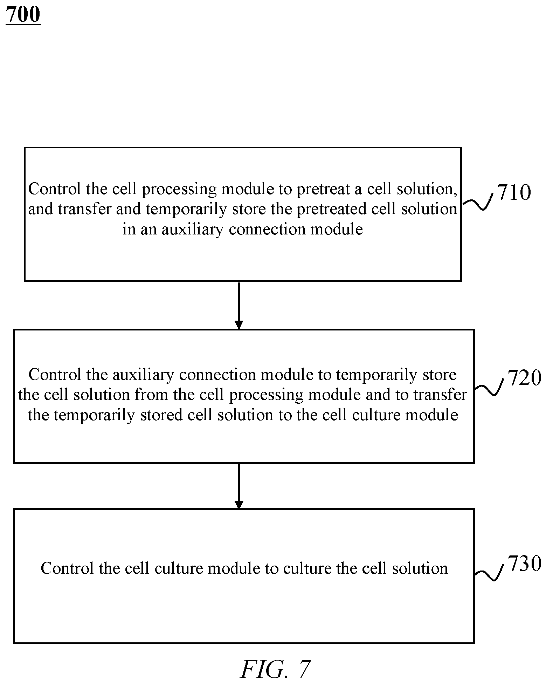

FIG. 7 is a flowchart of solution transfer by controlling an auxiliary connection module according to some embodiments of the present application. As shown in FIG. 7, a process 700 may include the following steps.

In step 710, sample parameter information of a cell solution temporarily stored in an auxiliary connection module is obtained.

In some embodiments, the operator or the processor may measure sample parameter information of the cell solution based on the cell solution in the sampling unit in the auxiliary connection module by controlling other detection devices. In some embodiments, the sample parameter information may include, but is not limited to, at least one of the volume, cell density and cell viability of the cell solution. In some embodiments, the sample parameter information may be obtained by means of cell quality inspection, such as cell counting and cell viability detection, etc.

In step 720, it is determined whether the sample parameter information is within a preset parameter information range.

In some embodiments, after obtaining the sample parameter information of the cell solution, the operator may input the sample parameter information of the cell solution into the control system for cell therapy. In some embodiments, the preset parameter information may include a preset cell viability range and/or a preset cell number range. The processor may determine whether the sample parameter information of the cell solution temporarily stored in the auxiliary connection module satisfies the preset parameter information. For example, the processor may determine whether the number of cells in the cell solution temporarily stored in the auxiliary connection module is within the preset cell number range, or the processor may determine whether the cell viability of the cell solution temporarily stored in the auxiliary connection module is within the preset cell viability range.

In step 730, if so, the auxiliary connection module is controlled to transfer the temporarily stored cell solution to the cell culture module.

In some embodiments, the processor receives a signal of the sample parameter information meeting a preset condition (i.e., the sample parameter information is within the preset parameter information range), and controls the auxiliary connection module to transfer the temporarily stored cell solution to the cell culture module. In some embodiments, if the sample parameter information does not satisfy the preset parameter information, the processor may send corresponding instructions, and control the system to send corresponding prompt information to enable the operator to pretreat the cell solution again.

It should be noted that the above description of the process 700 is only for illustration and explanation, and does not limit the scope of application of the present application. For those skilled in the art, various modifications and changes can be made to the process 700 under the guidance of the present application. However, these modifications and changes are still within the scope of the present application. For example, the preset parameter information is not limited to the preset cell viability range and preset cell number range described above, but may also be cell type information, etc.

The control system for cell therapy disclosed in the present application may have beneficial effects including but not limited to the following: (1) the cell processing module, the auxiliary connection modules and the cell culture module are connected in sequence to form a closed environment, which reduces the contact between the cell solution and the outside throughout the cell therapy process and reduces the possibility of contamination of the cell solution; (2) the auxiliary connection modules are used to transfer the cell solution, reducing operations such as manual transfer of the cell solution, which, on one hand, reduces the risk of contamination of the cell solution, and on the other hand, reduces the workload of an operator and improves the efficiency of cell culture; (3) the auxiliary connection modules can conveniently and quickly connect a different number of cell processing modules to the cell culture module, which achieves flexible and quick use, improves the efficiency of cell culture, and reduces the idle ratio of the control system; and (4) the processor controls the cell processing module, the auxiliary connection modules and the cell culture module, which implements automatic operation of the control system and reduces the work intensity of the operator.

The basic concepts have been described above, and it will be apparent to those skilled in the art that the foregoing detailed disclosure is intended to be exemplary only and does not constitute a limitation to this specification. Although not explicitly stated herein, those skilled in the art may make various modifications, improvements and amendments to this specification. Such modifications, improvements and amendments are suggested in this specification, and thus remain within the spirit and scope of the exemplary embodiments of this specification.

Meanwhile, this specification uses specific words to describe the embodiments of this specification. For example, "one embodiment", "an embodiment" and/or "some embodiments" mean a particular feature, structure or characteristic related to at least one embodiment of this specification. Hence, it should be emphasized and noted that "an embodiment" or "one embodiment" or "one alternative embodiment" mentioned in two or more different positions in this specification does not necessarily refer to the same embodiment. Furthermore, some features, structures or characteristics in one or more embodiments of this specification may be appropriately combined.

Furthermore, unless explicitly stated in the claims, the order of processing elements and sequences, the use of numbers and letters, or the use of other names described in this specification is not intended to limit the order of the process or method of this specification. Although some currently considered useful embodiments of the invention have been discussed in the above disclosure by way of various examples, it should be understood that such details are only for illustrative purposes, and that the appended claims are not limited to the disclosed embodiments; rather, the claims are intended to cover all amendments and equivalent combinations that are in line with the spirit and scope of the embodiments of this specification. For example, although the system components described above can be implemented by means of hardware devices, they may also be implemented only by software solutions, such as installing the described system on an existing server or mobile device.

Similarly, it should be noted that in order to simplify the presentation of the disclosure of this specification, and thereby help to understand one or more embodiments of the invention, in the previous descriptions of the embodiments of this specification, various features are sometimes combined into one embodiment, the accompanying drawings, or descriptions thereof. However, this method of disclosure does not imply that the subject of this specification requires more features than those mentioned in the claims. In fact, the embodiments have fewer features than all features of the individual embodiments disclosed above.

In some embodiments, numbers for describing components, properties and quantities are used. It should be understood that such numbers for describing the embodiments are modified by the modifiers "about", "approximately" or "substantially" in some examples. Unless otherwise stated, the modifiers "about", "approximately" or "substantially" indicate that a number is allowed to vary by ± 20%. Accordingly, in some embodiments, numerical parameters used in the specification and the claims are approximate values that may vary depending on desired features of individual embodiments. In some embodiments, with regard to the numerical parameters, the specified number of significant digits should be considered, and a general digit retaining method should be used. Although numerical ranges and parameters for determining the breadth of ranges in some embodiments of this specification are approximate values, such numerical values should be set as accurate as possible in feasible ranges in the specific embodiments.

Each patent, each patent application, each patent application publication and other materials, such as articles, books, instructions, publications, documents, etc. cited in this specification are hereby incorporated by reference into this specification in its entirety. Application history documents that are inconsistent with or conflict with the contents of this specification are excluded, and documents (currently or later appended to this specification) that limit the broadest scope of the claims of this specification are also excluded. It should be noted that if there is any inconsistency or conflict between the descriptions, definitions, and/or the use of terms in the accompanying materials of this specification and the contents of this specification, the descriptions, definitions, and/or the use of terms in this specification shall prevail.

Finally, it should be understood that the embodiments described in this specification are only intended to illustrate the principles of the embodiments of this specification. Other variations may also fall within the scope of this specification. Thus, by way of example but not limitation, alternative configurations of the embodiments of this specification can be considered consistent with the teachings of this specification. Accordingly, the embodiments of this specification are not limited to those explicitly presented and described in this specification.

## Claims

1. A control system for cell therapy, **characterized by** comprising: a cell processing module, an auxiliary connection module, and a cell culture module which are connected in sequence; and
a processor which is in communication connection with the cell processing module, the auxiliary connection module and the cell culture module,
wherein the processor controls the auxiliary connection module to temporarily store a cell solution of the cell processing module and to transfer the temporarily stored cell solution to the cell culture module.

2. The control system for cell therapy according to claim 1, **characterized in that** in response to sample parameter information of the cell solution temporarily stored in the auxiliary connection module satisfying preset parameter information, the processor transfers the temporarily stored cell solution to the cell culture module.

3. The control system for cell therapy according to claim 2, **characterized in that** the preset parameter information comprises a preset cell viability range and/or a preset cell number range.

4. The control system for cell therapy according to claim 1, **characterized in that** the cell processing module comprises at least one of a cell washing unit, a cell incubation unit and a cell sorting unit.

5. The control system for cell therapy according to claim 1, **characterized in that** the cell processing module comprises a cell sorting unit, the auxiliary connection module comprises a first auxiliary connection module, and the cell sorting unit is connected to the cell culture module by means of the first auxiliary connection module.

6. The control system for cell therapy according to claim 5, **characterized in that** the cell processing module comprises a cell incubation unit, the auxiliary connection module comprises a second auxiliary connection module, and the cell incubation unit is connected to the cell sorting unit by means of the second auxiliary connection module.

7. The control system for cell therapy according to claim 6, **characterized in that** the cell processing module comprises a cell washing unit, the auxiliary connection module comprises a third auxiliary connection module, and the cell washing unit is connected to the cell incubation unit by means of the third auxiliary connection module.

8. The control system for cell therapy according to claim 1, **characterized in that** a plurality of cell processing modules are provided, the plurality of cell processing modules are connected to the auxiliary connection module, and the plurality of cell processing modules are arranged in parallel.

9. The control system for cell therapy according to claim 1, **characterized in that** the cell culture module comprises at least one of a cell activation unit, a cell transduction unit and a cell expansion unit.

10. The control system for cell therapy according to claim 9, **characterized in that** the auxiliary connection module comprises a fourth auxiliary connection module, wherein the fourth auxiliary connection module is connected to the cell culture module.

11. The control system for cell therapy according to claim 10, **characterized in that** the system comprises a collection module, wherein the collection module is connected to the cell culture module by means of the fourth auxiliary connection module.

12. The control system for cell therapy according to claim 1, **characterized in that** a plurality of cell culture modules are provided, the plurality of cell culture modules are connected to the auxiliary connection module, and the plurality of cell culture modules are arranged in parallel.

13. The control system for cell therapy according to claim 1, **characterized in that** the auxiliary connection module comprises a power unit and a solution transfer pipeline assembly adapted to the power unit; and
the solution transfer pipeline assembly comprises a first solution transfer pipeline, wherein the first solution transfer pipeline comprises a first port and a second port, the power unit is adapted to the first solution transfer pipeline, the first port is connected to the cell processing module, and the second port is connected to the cell culture module.

14. The control system for cell therapy according to claim 13, **characterized in that** the auxiliary connection module comprises a temporary storage unit connected to the solution transfer pipeline assembly, the solution transfer pipeline assembly comprises a second solution transfer pipeline, and the second solution transfer pipeline communicates the temporary storage unit with the first solution transfer pipeline; a junction of the first solution transfer pipeline and the second solution transfer pipeline is located between the second port and the power unit; and
the solution transfer pipeline assembly comprises a third solution transfer pipeline, the third solution transfer pipeline communicates the second solution transfer pipeline with the first solution transfer pipeline, and a junction of the second solution transfer pipeline and the first solution transfer pipeline is located between the first port and the power unit.

15. The control system for cell therapy according to claim 14, **characterized in that** the first solution transfer pipeline between the first port and the power unit is provided with a first valve, the first solution transfer pipeline between the second port and the power unit is provided with a second valve, the second solution transfer pipeline is provided with a third valve, and the third solution transfer pipeline is provided with a fourth valve.

16. The control system for cell therapy according to claim 15, **characterized in that** the auxiliary connection module further comprises a sampling unit, the solution transfer pipeline assembly comprises a fourth solution transfer pipeline, the fourth solution transfer pipeline communicates the sampling unit with the first solution transfer pipeline, and a junction of the fourth solution transfer pipeline and the first solution transfer pipeline is located between the second port and the power unit, wherein the fourth solution transfer pipeline is provided with a fifth valve for controlling the connected and disconnected state of the fourth solution transfer pipeline.

17. A control method for a control system for cell therapy, **characterized in that** the control system for cell therapy comprises a cell processing module, an auxiliary connection module, and a cell culture module which are connected in sequence, and the control method comprises:
controlling the cell processing module to pretreat a cell solution, and transferring and temporarily storing the pretreated cell solution in the auxiliary connection module;
controlling the auxiliary connection module to temporarily store the cell solution of the cell processing module and to transfer the temporarily stored cell solution to the cell culture module; and
controlling the cell culture module to culture the cell solution.

18. The control system for cell therapy according to claim 17, **characterized in that** controlling the auxiliary connection module to temporarily store the cell solution of the cell processing module and to transfer the cell solution to the cell culture module comprises:
obtaining sample parameter information of the cell solution temporarily stored in the auxiliary connection module;
determining whether the sample parameter information is within a preset parameter information range; and
if so, controlling the auxiliary connection module to transfer the temporarily stored cell solution to the cell culture module.
